# EUROPÄISCHE PATENTSCHRIFT

(11) **EP 3 755 692 B1**
(45) Veröffentlichungstag und Bekanntmachung des Hinweises auf die Patenterteilung: **06.04.2022**
(21) Anmeldenummer: 19704324.3
(22) Anmeldetag: 11.02.2019
(51) Int. Cl.: C07D 295/023

(54) **VERFAHREN ZUR ABREICHERUNG VON 2-METHOXYETHANOL (MOE)**
METHOD FOR THE DEPLETION OF 2-METHOXYETHANOL (MOE)
PROCÉDÉ D'APPAUVRISSEMENT EN 2-MÉTHOXYÉTHANOL (MOE)

(30) Priorität: 22.02.2018 EP 18158113
(43) Veröffentlichungstag der Anmeldung: 30.12.2020
(73) Patentinhaber: BASF SE, 67056 Ludwigshafen am Rhein (DE)
(72) Erfinder: KOCH, Eva, 67056 Ludwigshafen (DE); BUSSMANN, Oliver, 67056 Ludwigshafen (DE); PFEFFINGER, Joachim, 67056 Ludwigshafen (DE); PASTRE, Joerg, 67056 Ludwigshafen (DE); MELDER, Johann-Peter, 67056 Ludwigshafen (DE)
(74) Vertreter: BASF IP Association
(86) Internationale Anmeldenummer: PCT/EP2019/053269
(87) Internationale Veröffentlichungsnummer: WO 2019/162120

(56) Entgegenhaltungen:
- WO-A1-2008/037589
- CN-A- 102 002 019
- CN-B- 102 206 196

## Beschreibung

Die Erfindung betrifft ein Verfahren zur Abreicherung von 2-Methoxyethanol aus einem Gemisch enthaltend überwiegend Morpholin (MO) (roh-Morpholin).

Die Trennung von 2-Methoxyethanol und Morpholin ist aufgrund der dicht beeinander liegenden Siedepunkte nur schwer möglich. Das Problem der Abreicherung von 2-Methoxyethanol aus einem überwiegend Morpholin enthaltenden Gemisch stellt sich insbesondere im Zusammenhang mit der Herstellung von Aminodiglykol (ADG) und Morpholin (MO) aus der Umsetzung von Diethylenglykol (DEG) und Ammoniak in Gegenwart von Wasserstoff und eines Heterogen-Hydrierkatalysators (Katalysator).

Aminodiglykol (ADG) [= 2-(2-Aminoethoxy)ethanol = 2,2'-Aminoethoxyethanol] und Morpholin finden unter anderem Verwendung als Lösungsmittel, Stabilisatoren, zur Synthese von ChelatBildnern, Kunstharzen, Arzneimitteln, Inhibitoren und grenzflächenaktiven Substanzen.

Zur Herstellung von ADG und MO sind in der Literatur zahlreiche Verfahren beschrieben.

Ullmann's Encyclopedia of Industrial Chemistry, 6th Edition, 2000 electronic release, Wiley-VCH Verlag, Rubrik 'cyclic amines' im Kapitel 'aliphatic amines' beschreibt die Synthese von ADG und MO durch Aminierung von DEG unter Wasserstoffdruck und in Gegenwart eines Kobalt- oder Nickelkatalysators (Zitate: EP-A-696 572 (BASF AG), DE-A-1 049 864) oder anderer Katalysatoren (Zitate: DE-A-3 002 342, DE-A-3 125 662 (BASF AG), US 3 155 657).

Die WO 2011/067199 A1 (BASF SE) betrifft u.a. die Umsetzung von Diethylenglycol (DEG) und Ammoniak in Gegenwart eines geträgerten, kupfer-, nickel- und kobalthaltigen Katalysators, dessen katalytisch aktive Masse vor seiner Reduktion mit Wasserstoff (H₂) sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält.

Die drei Patentanmeldungen WO 2008/037587 A1, WO 2008/037589 A1 und WO 2008/037590 A1 (alle BASF AG) betreffen Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) mit Ammoniak.

Die WO 2008/037659 A1 betrifft ein Verfahren zur Herstellung von ADG in Elektronik-Qualität.

Die CN 102002019 A beschreibt eine destillative Methode, um Methoxyethanol aus Morpholin zu entfernen. Hierzu wird Wasserdampf in die entsprechende Destillationskolonne eingespeist. Dabei wird ausgenutzt, dass Methoxyethanol mit Wasser ein Azeotrop bildet.

Die CN 104262173 A und CN 104262177 A beschreiben beide ein Verfahren zur Umsetzung von DEG mit Ammoniak. CN 104262177 A beschreibt ferner ein Verfahren zur Aufarbeitung des resultierenden Gemischs.

Der vorliegenden Erfindung lag die Aufgabe zugrunde, unter Überwindung eines Nachteils oder mehrerer Nachteile des Standes der Technik, ein verbessertes wirtschaftliches Verfahren zur Abreicherung von 2-Methoxyethanol aus einem überwiegend Morpholin enthaltenden Gemisch aufzufinden.

Demgemäß wurde ein Verfahren zur Abreicherung von 2-Methoxyethanol (MOE) aus einem Gemisch enthaltend überwiegend Morpholin (MO) (roh-Morpholin) gefunden, welches dadurch gekennzeichnet ist, dass roh-Morpholin in einer Destillationskolonne in Gegenwart einer Alkalimetallverbindung der allgemeinen Formel M⁺[RO⁻] (M⁺ bedeutet Alkalimetallkation und R bedeutet Wasserstoff (H), Methyl, Ethyl, Propyl, iso-Propyl, n-Butyl, iso-Butyl, sec-Butyl, oder tert-Butyl) destilliert wird, wobei MO sowie eine Verbindung der allgemeinen Formel R-OH abdestilliert werden und im Sumpf der Kolonne ein Alkalimetallmethoxyethanolat der allgemeinen Formel M⁺[MeOEtO⁻] anfällt.

Das erfindungsgemäße Verfahren wird in einer Destillationskolonne durchgeführt. Es kommen alle möglichen dem Fachmann bekannten Destillationskolonnen in Frage. Bevorzugt sind Packungskolonnen mit strukturierten Packungen oder Füllkörpern sowie Bodenkolonnen mit Böden wie Siebböden, Glockenböden oder Ventilböden.

Ohne die vorliegende Erfindung in irgendeiner Weise zu beschränken, ist davon auszugehen, dass des 2-Methoxyethanol durch das Hydroxid bzw. Alkoholat der allgemeinen Formel ROdeprotoniert wird und als Alkalimetallmethoxyethanolat im Sumpf der Kolonne gebunden wird. Aus dem besagten Hydroxid bzw. Alkoholat entsteht eine Verbindung der allgemeinen Formel R-OH, also entweder Wasser oder ein entsprechender Alkohol. Diese werden abdestilliert. Grundsätzlich ist erfindungsgemäß auch der Einsatz von mehreren Alkalimetallverbindungen möglich.

Das erfindungsgemäße Verfahren kann sowohl kontinuierlich als auch diskontinuierlich betrieben werden.

Der Fachmann wird den Kopfdruck und die Sumpftemperatur so einstellen, dass er die Verbindung der allgemeinen Formel R-OH sowie Morpholin abdestillieren kann.

Sofern nicht explizit etwas anderes angegeben ist, beziehen sich alle folgenden Druckangaben auf den Absolutdruck.

Der Kopfdruck in der Destillationskolonne beträgt bevorzugt 0,01 bis 12 bar, besonders bevorzugt 0,2 bis 5 bar und ganz besonders bevorzugt 0,5 bis 3 bar oder sogar 0,5 bis 2 bar.

Die Sumpftemperatur wird der Fachmann entsprechend so wählen, dass bei einem gegebenen Kopfdruck eine Verdampfung von R-OH beziehungsweise Morpholin erfolgt. Bei den oben genannten Bereichen für den Kopfdruck liegt die Sumpftemperatur typischerweise im Bereich von 50 bis 300 °C, besonders bevorzugt 80 bis 250 °C, ganz besonders bevorzugt 100 bis 200 °C.

Die Kopftemperatur entspricht dann im Wesentlichen dem Siedepunkt von R-OH beziehungsweise Morpholin bei dem jeweils eingestellten Kopfdruck.

Es wird davon ausgegangen, dass unter den gegebenen Destillationsbedingungen die Verbindung R-OH einen geringeren Siedepunkt als das Morpholin hat. Bei einem diskontinuierlichen Betrieb würde man also zunächst R-OH abdestillieren (die Kopftemperatur entspricht im Wesentlichen dem Siedepunkt von R-OH bei dem eingestellten Kopfdruck) und anschließend Morpholin (die Kopftemperatur steigt dann auf einen Wert, der im Wesentlichen dem Siedepunkt von Morpholin bei dem eingestellten Kopfdruck entspricht).

Bei einer kontinuierlichen Fahrweise ist es bevorzugt, dass roh-Morpholin und die Alkalimetallverbindung der Destillationskolonne zugeführt werden, R-OH und gegebenenfalls R'-OH (wie unten definiert) über Kopf und Morpholin (MO) über einen Seitenabzug abgeführt werden und über Sumpf Alkalimetallmethoxyethanolat ausgeschleust wird. Besonders bevorzugt befinden sich dabei die Zuführung der Alkalimetallverbindung und der Seitenabzug, an dem MO abgeführt wird, im Abtriebsteil der Destillationskolonne. Ganz besonders bevorzugt befindet sich hierbei die Zuführung der Alkalimetallverbindung über dem Seitenabzug, an dem Morpholin (MO) abgeführt wird. Die besagte Zuführung und der Seitenabzug befinden sich hierbei üblicherweise auf einander gegenüberliegenden Seiten der Destillationskolonne. In besonderem Maße bevorzugt wird die Position der Zuführung der Alkalimetallverbindung im Abtriebsteil der Kolonne so gewählt, dass dort keine signifikante Menge an Wasser mehr vorhanden ist. Hierunter ist insbesondere zu verstehen, dass der Wassergehalt dort ≤ 0,1 Gew.-%, besonders ≤ 0,05 Gew.-% oder ≤ 0,04 Gew.-%, z.B. 0,01 bis 0,03 Gew.-% beträgt. Der Wassergehalt lässt sich gemäß DIN EN 51777 (K. Fischer) bestimmen.

Grundsätzlich ist einer kontinuierlichen Reaktionsführung der Vorzug zu geben, weil sich hierdurch größere Mengen an reinem, also destilliertem, Morpholin (MO) herstellen lassen.

Das eingesetzte roh-Morpholin wird üblicherweise durch Umsetzung von Diethylenglykol (DEG) mit Ammoniak und anschließender destillativer Abtrennung von Ammoniak, Wasser, Aminodiglykol (ADG) und DEG aus dem Umsetzungsprodukt hergestellt. Eine solche Umsetzung und Abtrennung ist beispielsweise in der WO 2008/037587 A1, WO 2008/037589 A1 und WO 2008/037590 A1 (alle BASF AG) beschrieben.

Die Umsetzung von DEG und Ammoniak erfolgt üblicherweise in Gegenwart von Wasserstoff und eines Heterogen-Hydrierkatalysators (Katalysator).

In einer bevorzugten Ausführungsform (A) wird dabei ein Katalysator, enthaltend Cu, Ni, und Co auf Aluminiumoxid als Träger eingesetzt. Katalysatoren dieses Typs sind beispielsweise in WO 2011/067199 A1 (BASF SE) beschrieben.

In einem besonders bevorzugten Katalysator enthält die katalytisch aktive Masse des Katalysators vor der Behandlung mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO.

Insbesondere werden Katalysatoren eingesetzt, deren katalytisch aktive Masse vor deren Reduktion mit Wasserstoff im Bereich von
15 bis 80 Gew.-% sauerstoffhaltige Verbindungen des Aluminiums, berechnet als Al₂O₃,
1 bis 20 Gew.-% sauerstoffhaltige Verbindungen des Kupfers, berechnet als CuO,
5 bis 35 Gew.-% sauerstoffhaltige Verbindungen des Nickels, berechnet als NiO,
5 bis 35 Gew.-% sauerstoffhaltige Verbindungen des Kobalts, berechnet als CoO, und
0,2 bis 5,0 Gew.-% sauerstoffhaltige Verbindungen des Zinns, berechnet als SnO, enthält.

Die für die Umsetzung von Diethylenglykol (DEG) mit Ammoniak bevorzugte Temperatur im Reaktor liegt im Bereich von 170 bis 220°C. Bevorzugt ist eine isotherme Reaktorfahrweise. Der für die Umsetzung von Diethylenglykol (DEG) mit Ammoniak bevorzugte Druck liegt im Bereich von 100 bis 300 bar.

Bevorzugt wird die Umsetzung von Diethylenglykol (DEG) mit Ammoniak in Gegenwart von Wasserstoff durchgeführt. Der Wasserstoff wird bevorzugt über einen Hochdruckabscheider als Kreisgas in den Reaktor zurückgeführt.

Das Molverhältnis Ammoniak zu DEG liegt bevorzugt im Bereich von 4 bis 10.

Der DEG-Umsatz liegt bevorzugt im Bereich von 40 bis 90 %, bevorzugt 50 bis 80 %, besonders bevorzugt 50 bis 75% oder sogar 50 bis 70%.

Die Katalysatorbelastung liegt im Allgemeinen im Bereich von 0,05 bis 5, bevorzugt 0,1 bis 2 kg Diethylenglykol (DEG) pro Liter Katalysator (Schüttvolumen) und Stunde.

Besonders bevorzugt erfolgt die Herstellung des im erfindungsgemäßen Verfahren eingesetzten Gemisches gemäß der WO 2011/067199 A1 (BASF SE).

In einer anderen bevorzugten Ausführungsform (B) wird Katalysator, enthaltend Cu und Ni auf Aluminiumoxid als Träger, wie insbesondere in EP-A-70 397 (BASF AG) beschrieben, eingesetzt. Katalysatoren dieses Typs sind auch in EP-A-514 692 und EP-A-167 872 (beide BASF AG) beschrieben.

In einem hierbei besonders bevorzugten Katalysator enthält die katalytisch aktive Masse des Katalysators vor der Behandlung mit Wasserstoff im Bereich von 25 bis 65 Gew.-% Aluminiumoxid (Al₂O₃), 30 bis 60 Gew.-%, sauerstoffhaltige Verbindungen des Kupfers, berechnet als CuO, und 5 bis 15 Gew.-%, sauerstoffhaltige Verbindungen des Nickels, berechnet als NiO.

Die für die Umsetzung von Diethylenglykol (DEG) mit Ammoniak bevorzugte Temperatur im Reaktor liegt hierbei im Bereich von 190-235°C. Bevorzugt ist eine isotherme Reaktorfahrweise. Der für die Umsetzung von Diethylenglykol (DEG) mit Ammoniak bevorzugte Druck liegt im Bereich von 20 bis 30 bar.

Das Molverhältnis Ammoniak zu DEG liegt bevorzugt im Bereich von 1:1 bis 50:1.

Der DEG-Umsatz liegt bevorzugt im Bereich von 80 bis 98 %.

Die Katalysatorbelastung liegt im Allgemeinen im Bereich von 0,01 bis 2, bevorzugt 0,05 bis 0,5 kg Diethylenglykol (DEG) pro Liter Katalysator (Schüttvolumen) und Stunde.

Die Aufarbeitung eines gemäß den Ausführungsformen (A) und (B) erhältlichen Gemischs, insbesondere die Abtrennung von Ammoniak, Wasser, Aminodiglykol (ADG) und DEG aus dem Umsetzungsprodukt, um roh-Morpholin zu erhalten, ist beispielsweise in der oben bereits genannten Anmeldung WO 2008/037587 A1 (BASF AG) beschrieben.

Bei der Umsetzung von Diethylenglykol (DEG) mit Ammoniak entstehen neben Aminodiglykol (ADG), Morpholin (MO) und dem (unerwünschten) Nebenprodukt 2-Methoxyethanol (MOE), weitere Wertprodukte wie beispielsweise N-Ethyl-Morpholin (E-MO) und 1,2-Ethylendiamin (EDA).

Das roh-Morpholin enthält bevorzugt > 50 Gew.-%, bevorzugt > 70 Gew.-%, besonders bevorzugt > 85 Gew.-% Morpholin (MO). Ferner enthält das roh-Morphollin üblicherweise < 10 Gew.-%, bevorzugt < 5 Gew.-% und besonders bevorzugt 0,1 bis 5 Gew.-%, beispielsweise 0,1 bis 1,5 Gew.-% Methoxyethanol (MOE). Der Wassergehalt liegt bevorzugt bei ≤ 1 Gew.-%, besonders bevorzugt 0,001 bis 1 Gew.-%.

Bevorzugt enthält das roh-Morpholin > 85 Gew.-% Morpholin (MO) und 0,1 bis 5 Gew.-% Methoxyethanol (MOE). Besonders bevorzugt enthält das roh-Morpholin > 85 Gew.-% Morpholin (MO), 0,1 bis 5 Gew.-% Methoxyethanol (MOE) und ≤ 1 Gew.-%, insbesondere 0,001 bis 1 Gew.-% Wasser.

Die genaue Zusammensetzung des roh-Morpholins hängt insbesondere davon ab, wie es hergestellt wurde und wie das erhaltene Reaktionsprodukt (destillativ) aufgereinigt wurde.

Das roh-Morpholin hat insbesondere
- eine Reinheit von 85 bis 99 Gew.-%, bevorzugt 85 bis 97 Gew.-%,
- einen Gehalt an 1,2-Ethylendiamin (EDA) von ≤ 10 Gew.-%, bevorzugt ≤ 8 Gew.-%,
- einen Gehalt an N-Ethyl-Morpholin (E-MO) von 0,01 bis 0,25 Gew.-%, bevorzugt 0,01 bis 0,15 Gew.-%,
- einen Gehalt an 2-Methoxyethanol (MOE) von 0,1 bis 5 Gew.-%, bevorzugt 0,1 bis 4 Gew.-%,
- einen Gehalt an Wasser von ≤ 1 Gew.-%, bevorzugt 0,001 bis 1 Gew.-%.

Alle gewichtsprozentualen Angaben betreffend das roh-Morpholin beziehen sich auf die Gesamtmasse des roh-Morpholins vor Zugabe der Alkalimetallverbindung.

Enthält das roh-Morpholin beispielsweise noch signifikante Mengen an 1,2-Ethylendiamin, so lässt sich dies im erfindungsgemäßen Verfahren über Kopf entfernen.

Erfindungsgemäß wurde erkannt, dass jede noch so geringe Menge der Alkalimetallverbindung prinzipiell zu einer Verringerung des Methoxyethanols (MOE) im destillierten Morpholin (MO) führt. Bevorzugt wird die Alkalimetallverbindung in der 0,1- bis 20-fachen (beispielsweise 0,2 bis 10-fachen oder 0,1 bis 5-fachen), besonders bevorzugt 0,5 bis 5-fachen (beispielsweise der 1 bis 5-fachen oder 1,1 bis 5-fachen), besonders bevorzugt 1,3- bis 3-fachen (beispielsweise der 1,4 bis 2,5-fachen oder 1,5- bis 2-fachen) molaren Menge bezogen auf
(a) wenn ein Alkalimetallhydroxid (Alkalimetallverbindung in der R Wasserstoff (H) bedeutet) eingesetzt wird, das im roh-Morpholin enthaltende MOE (2-Methoxyethanol),
(b) wenn ein Alkalimetallalkoholat (Alkalimetallverbindung in der R nicht Wasserstoff (H) bedeutet) eingesetzt wird,
   ■ bei einer diskontinuierlichen Fahrweise, das im roh-Morpholin enthaltende MOE (2-Methoxyethanol) und gegebenenfalls im roh-Morpholin vorhandenes Wasser, oder
   ■ bei einer kontinuierlichen Fahrweise, das im roh-Morpholin enthaltende MOE (2-Methoxyethanol),
   eingesetzt wird.

Handelt es sich bei der Alkalimetallverbindung nicht um ein Alkalimetallalkoholat, sondern ein Alkalimetallhydroxid, so bezieht sich die molare Menge ausschließlich auf MOE ohne dass gegebenenfalls im roh-Morpholin vorhandenes Wasser berücksichtigt wird. Ohne den Gegenstand der Erfindung hierdurch in irgendeiner Weise zu beschränken, liegt dieser Unterscheidung folgende Überlegung zugrunde. Im Falle des Einsatzes eines Alkalimetallalkoholats erfolgt durch gegebenenfalls vorhandenes Wasser eine Protonierung des Alkoholats. Letzteres ist dann nicht mehr in der Lage, ein MOE Molekül zu deprotonieren. Im Falle eines Alkalimetallhydroxids spielt dies keine Rolle, weil durch eine Protonierung von OH- durch gegebenenfalls vorhandenes Wasser formal keine Verringerung der Stoffmenge an OH- eintritt.

Wird ein Alkalimetallalkoholat eingesetzt ist üblicherweise zwischen einer diskontinuierlichen und kontinuierlichen Fahrweise zu unterscheiden. Bei einer diskontinuierlichen Fahrweise ist aus den oben genannte Gründen gegebenenfalls im roh-Morpholin vorhandenes Wasser zu berücksichtigen. Bei einer kontinuierlichen Fahrweise wird die Position der Zulaufstelle bevorzugt gerade so gewählt, dass dort keine signifikante Menge an Wasser mehr vorhanden ist (siehe oben). Diese Möglichkeit ergibt sich letztlich daraus, dass unter den gegebenen Destillationsbedingungen Wasser ein Leichtsieder ist, der über Kopf abgetrennt wird, d.h. sich im Verstärkerteil der Destillationskolonne anreichert. Enthält das eingesetzte roh-Morpholin also noch geringe Mengen an Wasser, so ist der Gehalt an Wasser im Abtriebsteil der Kolonne entsprechend geringer als im roh-Morpholin.

M⁺ (Alkalimetallkation) bedeutet bevorzugt Li⁺, Na⁺, oder K⁺, besonders bevorzugt Na⁺ oder K⁺.

R bedeutet bevorzugt Methyl, Ethyl, Propyl, iso-Propyl, n-Butyl, iso-Butyl, sec-Butyl, oder tert-Butyl. D.h. der Einsatz eines Alkalimetallalkoholats (Alkalimetallverbindung der allgemeinen Formel M⁺[RO⁻], in der R nicht Wasserstoff (H) bedeutet) ist gegenüber dem Einsatz eines Alkalimetallhydroxids (Alkalimetallverbindung der allgemeinen Formel M⁺[RO⁻], in der R Wasserstoff (H) bedeutet) bevorzugt. Besonders bevorzugt bedeute R Methyl, Ethyl, Propyl oder iso-Propyl und ganz besonders bevorzugt Methyl oder Ethyl.

Die Alkalimetallverbindung ist bevorzugt ausgewählt aus der Gruppe bestehend aus Lithiumhydroxid, Natriumhydroxid, Kaliumhydroxid, Lithiummethanolat, Natriummethanolat, Kaliummethanolat, Lithiumethanoloat, Natriumethanoloat, Kaliumethanoloat, Lithiumpropanolat, Natriumpropanolat, Kaliumpropanolat, Lithiumisopropanolat, Natriumisopropanolat, Kaliumisopropanolat, Lithium-n-butanolat, Natrium-n-butanolat, Kalium-n-butanolat, Lithium-iso-butanolat, Natrium-iso-butanolat, Kalium-iso-butanolat, Lithium-sec-butanolat, Natrium-sec-butanolat, Kalium-sec-butanolat, Lithium-tert-butanolat, Natrium-tert-butanolat, und Kalium-tert-butanolat.

Die Alkalimetallverbindung ist besonders bevorzugt ausgewählt aus der Gruppe bestehend aus Lithiummethanolat, Natriummethanolat, Kaliummethanolat, Lithiumethanoloat, Natriumethanoloat, Kaliumethanoloat, Lithiumpropanolat, Natriumpropanolat, Kaliumpropanolat, Lithiumisopropanolat, Natriumisopropanolat, Kaliumisopropanolat, Lithium-n-butanolat, Natrium-n-butanolat, Kalium-n-butanolat, Lithium-iso-butanolat, Natrium-iso-butanolat, Kalium-iso-butanolat, Lithium-sec-butanolat, Natrium-sec-butanolat, Kalium-sec-butanolat, Lithium-tert-butanolat, Natrium-tert-butanolat, und Kalium-tert-butanolat.

Die Alkalimetallverbindung ist ganz besonders bevorzugt ausgewählt aus der Gruppe bestehend aus Lithiummethanolat, Natriummethanolat, Kaliummethanolat, Lithiumethanoloat, Natriumethanoloat, Kaliumethanoloat, Lithiumpropanolat, Natriumpropanolat, Kaliumpropanolat, Lithiumisopropanolat, Natriumisopropanolat, Kaliumisopropanolat.

In einer bevorzugten Ausführungsform des erfindungsgemäßen Verfahrens wird die Alkalimetallverbindung als Lösung in einem Alkohol der allgemeinen Formel R'-OH eingesetzt, in der R' Methyl, Ethyl, Propyl, iso-Propyl, n-Butyl, iso-Butyl, sec-Butyl, oder tert-Butyl bedeutet und R'-OH ebenfalls abdestilliert wird. Wie oben bereits für R-OH gesagt, wird auch R'-OH unter den gegebenen Destillationsbedingungen üblicherweise einen geringeren Siedepunkt als das Morpholin haben. Das oben gesagte gilt daher analog für das Abdestillieren von R'-OH. Entspricht R' dem Rest R, wird erfindungsgemäß ein Alkohol abdestilliert. Entspricht der Rest R' nicht dem Rest R, werden zwei Alkohole abdestilliert.

R' bedeutet bevorzugt Methyl, Ethyl, Propyl oder iso-Propyl und besonders bevorzugt Methyl oder Ethyl.

Für den (bevorzugten) Fall, dass ein Alkalimetallalkoholat eingesetzt wird, entspricht der Rest R' bevorzugt dem Rest R. Dies entspricht der üblichen Herstellung eines Alkalimetallalkoholats, wobei das entsprechende Alkalimetall (M) in dem gewünschten Alkohol unter Freisetzung von Wasserstoff zum Alkalimetallkation (M⁺) oxidiert wird.

Bevorzugt beträgt der Gehalt der Alkalimetallverbindung in der Lösung 1 bis 50 Gew.-%, besonders bevorzugt 10 bis 40 Gew.-%, ganz besonders bevorzugt 15 bis 40 Gew.-% (bezogen auf die Gesamtmasse der Lösung). Diese Angaben beziehen sich auf die Lösung der Alkalimetallverbindung vor ihrer Zugabe in den Sumpf.

In einer besonders bevorzugten Ausführungsform des erfindungsgemäßen Verfahrens wird als Alkalimetallverbindung Natriummethylat (20 bis 40 Gew.-%ig in Methanol) oder Kaliumethylat (15 bis 35 Gew.-%ig in Ethanol) eingesetzt.

Das mithilfe des erfindungsgemäßen Verfahrens hergestellte (destillierte) Morpholin (MO) hat bevorzugt eine Reinheit von > 98,5 Gew.-%, bevorzugt 99,0 Gew.-%.

Ferner liegt der Gehalt an Methoxyethanol (MOE) bevorzugt bei < 0,1 Gew.-%, insbesondere < 0,05 Gew.-%, besonders < 0,03 Gew.-% oder sogar < 0,01 Gew.-%.

Bevorzugt liegt die Reinheit bei > 98,5 Gew.-% und der Gehalt an Methoxyethanol (MOE) bei < 0,1 Gew.-%.

Das so hergestellte (destillierte) Morpholin (MO) hat insbesondere
- eine Reinheit von > 98,5 Gew.-%, bevorzugt > 99 Gew.-%, besonders bevorzugt > 99,3 Gew.-%,
- einen Gehalt an 2-Methoxyethanol (MOE) von < 0,3 Gew.-%, insbesondere < 0,1 Gew.-%, besonders < 0,03 Gew.-% oder sogar < 0,01 Gew.-%,
- einen Gehalt an N-Ethylmorpholin (E-MO) von ≤ 0,20 Gew.-%, besonders ≤ 0,10 Gew.-%, z.B. 0,01 bis 0,08 Gew.-%,
- einen Gehalt an 1,2-Ethylendiamin (1,2-EDA) von ≤ 0,30 Gew.-%, besonders ≤ 0,20 Gew.-%, z.B. 0,05 bis 0,15 Gew.-%, und
- einen Gehalt an Wasser von ≤ 0,1 Gew.-%, besonders ≤ 0,05 Gew.-% oder ≤ 0,04 Gew.-%, z.B. 0,01 bis 0,03 Gew.-%.

Des Weiteren kann das hergestellte Morpholin (MO) einen Alkohol der Formel R-OH und gegebenenfalls der Formel R'-OH enthalten mit einem Gehalt von üblicherweise ≤ 1 Gew.-%, bevorzugt ≤ 0,6 Gew.-%, besonders bevorzugt ≤ 0,5 Gew.-%.

Alle gewichtsprozentualen Angaben betreffend das destillierte Morpholin beziehen sich auf die Gesamtmasse des nach der erfindungsgemäßen Destillation erhaltenen Destillats, d.h. Morpholin inklusive etwaige Nebenkomponenten wie MOE, E-MO etc.

Im roh-Morpholin sowie im Wertprodukt Morpholin lässt sich der Gehalt an Morpholin, 2-Methoxyethanol, N-Ethylmorpholin, 1,2-Ethylendiamin und Alkohol R-OH bzw. R'-OH mittels GC bestimmen. Die GC-Bedingungen sind dabei gemäß den entsprechenden Angaben im Beispielteil der Anmeldung zu wählen. Der Gehalt an Wasser lässt sich gemäß DIN EN 51777 (K. Fischer) bestimmen.

Die folgenden Beispiele dienen der Verdeutlichung der Erfindung, ohne sie in irgendeiner Weise zu beschränken.

### BEISPIELE

Die APHA-Farbzahl wurde gemäß DIN EN 1557 bestimmt.

Der Wassergehalt wurde gemäß DIN EN 51777 (K. Fischer) bestimmt.

GC-Bedingungen: 30 m Db-1; Temperaturprogramm mit 60 °C Anfangstemperatur, 4 °C/min. Heizrate, 190°C Endtemperatur.

Beispiel 1 (Destillation von Morpholin (MO) in Gegenwart von Natriummethylat)

Kolonnentyp:
1,0m Sulzer DX-Packung,
Durchmesser 42 mm (A=13,85 cm²)
Theoretische Bodenzahl: 25 - 30

1503 g roh-Morpholin (enthaltend 0,048 Gew.-% Wasser und 0,4 Gew.-% MOE) wurden im Reaktor vorgelegt und unter Rühren mit 32g 30 %iger Natriummethylat-Lösung (entspricht der 1,5-fachen molaren Menge bezogen auf MOE und Wasser im roh-Morpholin) versetzt. Das resultierende Gemisch wird als Einsatzstoff bezeichnet. Es wurde bei Normaldruck (ND = 1,013 bar) bis zum Sieden erhitzt und die Kolonne für 60 Minuten in den Rücklauf gestellt. Mit einem Rücklaufverhältnis (RLV) von 10 wurde Methanol entfernt. Nach Erreichen einer Kopftemperatur von 128 °C wurde in den Hauptlauf umgestellt. Die beiden Fraktionen wurden mittels Gaschromatographie (GC) analysiert.

**Tabelle 1: Destillationsergebnisse für Natriummethylat**

| | Einsatzstoff | Fraktion 1 | Fraktion 2 |
|---|---|---|---|
| Masse/g | 1535 | 107 | 1276 |
| Druck (Kopf) | | ND | ND |
| Temperatur/°C (Sumpf) | | 129 | 129 - 137 |
| Temperatur/°C (Kopf) | | 65-128 | 128 |
| Temperatur/°C (Kühler) | | 20 | 20 |
| APHA-Farbzahl | | 10 | 11 |
| Wasser | 0,00 | 0,07 | 0,02 |
| Methanol /GC-FI. % | 1,47 | 19,70 | 0,05 |
| 2-Methoxyethanol /GC-FI. % | 0,39 | 0,00 | 0,00 |
| 1,2-Ethylendiamin /GC-FI.-% | 0,00 | 0,57 | 0,00 |
| Morpholin/ GC-FI.% | 97,51 | 79,71 | 99,96 |
| Morpholin/g | 1497 | 85 | 1275 |

Ergebnis:
Mit Hilfe von Natriummethylat konnte das Methoxyethanol während der gesamten Destillation im Sumpf gebunden werden. Nach der Methanol-Entfernung war Morpholin mit hoher Reinheit und geringer Farbzahl (Fraktion 2) destillierbar. Die Destillationsausbeute für Fraktion 2 betrug 85%.

### Beispiel 2 (Destillation von Morpholin (MO) in Gegenwart von Kaliumethylat

Es wurde derselbe Kolonnentyp wie in Beispiel 1 eingesetzt.

1683 g roh-Morpholin (enthaltend 0,023 Gew.-% Wasser und 0,4 Gew.-% MOE) wurden in einem Reaktor vorgelegt und unter Rühren mit 58g einer 24 %igen Kaliumethylat-Lösung (entspricht der 1,5-fachen molaren Menge bezogen auf MOE und Wasser im roh-Morpholin) versetzt. Das resultierende Gemisch wird als Einsatzstoff bezeichnet. Anschließend wurde bei Normaldruck (ND) bis zum Sieden erhitz und die Kolonne für 60 Minuten in den Rücklauf gestellt. Mit einem Rücklaufverhältnis (RLV) von 10 bis 15 wurde Ethanol entfernt. Nach Erreichen einer Kopftemperatur von 128 °C wurde in den Hauptlauf. Die Fraktionen wurden mittels GC analysiert.

**Tabelle 2: Destillationsergebnisse für Kaliumethylat**

| | Einsatzstoff | Fraktion 1 | Fraktion 2 | Fraktion 3 |
|---|---|---|---|---|
| Masse/g | 1741 | 268 | 160 | 1193 |
| Druck (Kopf) | | ND | ND | ND |
| Temperatur/°C (Sumpf) | | 128-129 | 129 | 129 |
| Temperatur/°C (Kopf) | | 77-127 | 127-128 | 128 |
| Temperatur/°C (Kühler) | | 20 | 20 | 20 |
| APHA-Farbzahl | | | | 11 |
| Wasser | 0,00 | | | 0,016 |
| Ethanol /GC-FI. % | 2,53 | 17,72 | 0,77 | 0,10 |
| 2-Methoxyethanol /GC-FI. % | 0,39 | 0,00 | 0,00 | 0,00 |
| 1,2-Ethylendiamin /GC-FI.-% | 0,00 | 0,00 | 0,00 | 0,00 |
| Morpholin/ GC-FI.% | 96,29 | 80,44 | 98,93 | 99,85 |
| Unbekannte/ GC-FI.% | 0,00 | 0,04 | 0,27 | 0,04 |
| Morpholin/g | 1677 | 216 | 158 | 1191 |

Ergebnis:
Mit Hilfe von Kaliumethylat konnte das Methoxyethanol während der gesamten Destillation im Sumpf gebunden werden. Nach der Ethanol-Entfernung war Morpholin mit hoher Reinheit und geringer Farbzahl destillierbar. Die Destillationsausbeute für Fraktion 3 betrug 71%.

### Beispiel 3 (Destillation von Morpholin (MO) in Gegenwart von Natriumhydroxid)

### Es wurde derselbe Kolonnentyp wie in Beispiel 1 eingesetzt.

1682 g roh-Morpholin (enthaltend 0,4 Gew.-% MOE und 0,00 Gew.-% Wasser) wurden in einem Reaktor vorgelegt und unter Rühren mit 28,4 g einer 23,3%igen methanolischen NaOH-Lösung (entspricht der 1,86-fachen molaren Menge bezogen auf MOE im roh-Morpholin) versetzt. Anschließend wurde die Mischung bei ND bis zum Sieden erhitzt und Kolonne für 60 Minuten in den Rücklauf gestellt. Mit einem RLV von 15 wurde Methanol entfernt. Ab einer Kopftemperatur von 128 °C wurde in den Hauptlauf umgestellt. Der Kopfkühler wurde bei einer Temperatur von 75 °C betrieben (Teilkondensator).

**Tabelle 3: Destillationsergebnisse für Natriumhydroxid**

| | Einsatzstoff | Fraktion 1 | Fraktion 2 | Fraktion 3 |
|---|---|---|---|---|
| Masse/g | 1710 | 185 | 147 | 1261 |
| Druck (Kopf) | | ND | ND | ND |
| Temperatur/°C (Sumpf) | | 130 | 129 | 129-145 |
| Temperatur/°C (Kopf) | | 114-128 | 128 | 128 |
| Temperatur/°C (Kühler) | | 75 | 75 | 75 |
| APHA-Farbzahl | | | | 0,04 |
| Wasser | 0,00 | | | 0,016 |
| Methanol /GC-FI. % | 1,27 | 13,33 | 0,51 | 0,10 |
| 2-Methoxyethanol /GC-FI. % | 0,39 | 0,00 | 0,00 | 0,03 |
| 1,2-Ethylendiamin /GC-FI.-% | 0,00 | 0,00 | 0,00 | 0,00 |
| Morpholin/ GC-FI.% | 97,95 | 86,47 | 99,38 | 99,83 |
| Unbekannte/ GC-FI.% | 0,00 | 0,11 | 0,12 | 0,00 |
| Morpholin/g | 1675 | 160 | 146 | 1259 |

Ergebnis:
Mit Hilfe von NaOH konnte das Methoxyethanol während der gesamten Destillation größtenteils im Sumpf gebunden werden. Nach der Methanol-Entfernung (Fraktionen 1 und 2) war Morpholin mit hoher Reinheit und geringer Farbzahl (Fraktion 3) destillierbar. Die Destillationsausbeute für Fraktion 3 betrug 75%.

### Beispiel 4 (Kontinuierliche bzw. semi-batch-Destillation von Morpholin (MO) in Gegenwart von Natriummethylat)

Kolonnentyp:
1,5 m Sulzer DX-Packung,
Durchmesser 30 mm
Theoretische Bodenzahl: 30 - 45

Kontinuierliche Fahrweise (Fraktion 1):
500 g roh-Morpholin (enthaltend 0,02 Gew.-% Wasser und 0,26 Gew.-% MOE) wurden im Reaktor vorgelegt und unter Rühren mit 4,8 g 30%iger Natriummethylat-Lösung versetzt. Anschließend wurde die resultierende Mischung bei ND bis zum Sieden erhitzt und die Kolonne für 30 Minuten in den Rücklauf gestellt. Mit einem RLV von 3 wurde Morpholin destilliert. Zeitgleich wurde MOE-haltiges Morpholin und Natriummethylat in den Sumpf der Destillation dosiert. Um Methanol selektiv zu entfernen, wurde der Kopfkühler bei ca. 80°C als Teilkondensator betrieben. Dabei wurde die Fraktion 1 kondensiert, wobei der überwiegende Teil des Methanols in der Gasphase verblieb. Das Methanol wurde dann ein einem zweiten, nachgestalteten Kondensator kondensiert. Nach 26 h wurde der Versuch abgebrochen. Die Ergebnisse zeigen eine deutliche Abreicherung von Methoxyethanol in der Fraktion 1.

Semi-batch Fahrweise (Fraktion 2 und 3):
Im Reaktor wurden 730g roh Morpholin (enthaltend 0,02 Gew.-% Wasser und 0,26 Gew.-% MOE) vorgelegt und unter Rühren mit 48,5 g 30%iger Natriummethylat-Lösung versetzt. Anschließend wurde die resultierende Mischung bei ND bis zum Sieden erhitzt und die Kolonne für 30 Minuten in den Rücklauf gestellt. Mit einem RLV von 3 wurde Morpholin destilliert. Zeitgleich wurde MOE-haltiges Morpholin in den Sumpf der Destillation dosiert. Das Methanol wurde genau wie bei der kontinuierlichen Fahrweise entfernt. Die Ergebnisse zeigen eine deutliche Abreicherung von Methoxyethanol in der Fraktion 2.

Nach 8 h wurde die Zulaufmenge des Morpholins von 300 auf 400 g/h erhöht und das gereinigte Morpholin als Fraktion 3 gesammelt. Nach 9 h wurde der Versuch beendet. In Fraktion 3 ist verglichen mit Fraktion 1 und 2 ein erhöhter Gehalt an Methoxyethanol zu verzeichnen. Dies hängt damit zusammen, dass kein neues Natriummethylat mehr hinzugegeben wurde, sprich das Natriummethylat nicht mehr in ausreichender Menge zur Verfügung stand, um das Methoxyethanol quantitativ im Sumpf zu binden. Dies belegt, dass die Bindung des Methoxyethanols im Sumpf auf das Vorhandensein des Natriummethylats zurückzuführen ist.

| | Einsatzstoff | Fraktion 1 | Fraktion 2 | Fraktion 3 |
|---|---|---|---|---|
| Masse/g | 12805 g roh-MO und 128 g Na-Methylat | 185 | 147 | 1261 |
| Druck (Kopf) | | ND | ND | ND |
| Temperatur/°C (Sumpf) | | 129 - 130 | 129 - 130 | 130 |
| Temperatur/°C (Kopf) | | 126 | 126 | 126-127 |
| Temperatur/°C Teilkondensator | | 75-80 | 80 | 80 |
| Temperatur/°C (Kühler) | | 20 | 20 | 20 |
| APHA-Farbzahl | 5 | 10 | 11 | 10 |
| Wasser | 0,020 | 0,065 | 0,006 | 0,002 |
| Methanol /GC-FI. % | 0,560 | 0,492 | 0,099 | 0,027 |
| 2-Methoxyethanol /GC-FI. % | 0,261 | 0,008 | 0,003 | 0,023 |
| 1,2-Ethylendiamin /GC-FI.-% | 0,00 | 0,036 | 0,018 | 0,011 |
| Morpholin/ GC-FI.% | 98,939 | 99,453 | 99,870 | 99,932 |
| Morpholin/g | 12669 | 5444 | 2534 | 2884 |

Die in der Spalte "Einsatzstoffe" angegebenen Werte beziehen sich auf den Zulaufstrom in die Kolonne Die Destillationsausbeute für die Fraktionen 1 bis 3 betrug 86%.

## Patentansprüche

1. Verfahren zur Abreicherung von 2-Methoxyethanol (MOE) aus einem Gemisch enthaltend überwiegend Morpholin (MO) (roh-Morpholin), **dadurch gekennzeichnet, dass** roh-Morpholin in einer Destillationskolonne in Gegenwart einer Alkalimetallverbindung der allgemeinen Formel M⁺[RO⁻] (M⁺ bedeutet Alkalimetallkation und R bedeutet Wasserstoff (H), Methyl, Ethyl, Propyl, iso-Propyl, n-Butyl, iso-Butyl, sec-Butyl, oder tert-Butyl) destilliert wird, wobei MO sowie eine Verbindung der allgemeinen Formel R-OH abdestilliert werden und im Sumpf der Kolonne ein Alkalimetallmethoxyethanolat der allgemeinen Formel M⁺[MeOEtO⁻] anfällt.

2. Verfahren nach Anspruch 1, **dadurch gekennzeichnet, dass** die Alkalimetallverbindung in der 0,1 bis 5-fachen molaren Menge bezogen auf
(a) wenn ein Alkalimetallhydroxid (Alkalimetallverbindung in der R Wasserstoff (H) bedeutet) eingesetzt wird, das im roh-Morpholin enthaltende MOE (2-Methoxyethanol),
(b) wenn ein Alkalimetallalkoholat (Alkalimetallverbindung in der R nicht Wasserstoff (H) bedeutet) eingesetzt wird,
■ bei einer diskontinuierlichen Fahrweise, das im roh-Morpholin enthaltende MOE (2-Methoxyethanol) und gegebenenfalls im roh-Morpholin vorhandenes Wasser, oder
■ bei einer kontinuierlichen Fahrweise, das im roh-Morpholin enthaltende MOE (2-Methoxyethanol),
eingesetzt wird.

3. Verfahren nach einem der vorhergehenden Ansprüche, **dadurch gekennzeichnet, dass** M⁺ Li⁺, Na⁺ oder K⁺ bedeutet.

4. Verfahren nach einem der vorhergehenden Ansprüche, **dadurch gekennzeichnet, dass** die Alkalimetallverbindung ausgewählt ist aus der Gruppe bestehen aus Lithiumhydroxid, Natriumhydroxid, Kaliumhydroxid, Lithiummethanolat, Natriummethanolat, Kaliummethanolat, Lithiumethanoloat, Natriumethanoloat, Kaliumethanoloat, Lithiumpropanolat, Natriumpropanolat, Kaliumpropanolat, Lithiumisopropanolat, Natriumisopropanolat, Kaliumisopropanolat, Lithium-n-butanolat, Natrium-n-butanolat, Kalium-n-butanolat, Lithium-iso-butanolat, Natrium-iso-butanolat, Kalium-iso-butanolat, Lithium-sec-butanolat, Natrium-sec-butanolat, Kalium-sec-butanolat, Lithium-tert-butanolat, Natrium-tert-butanolat, und Kalium-tert-butanolat.

5. Verfahren nach einem der vorhergehenden Ansprüche, **dadurch gekennzeichnet, dass** die Alkalimetallverbindung als Lösung in einem Alkohol der allgemeinen Formel R'-OH eingesetzt wird, in der R' Methyl, Ethyl, Propyl, iso-Propyl, n-Butyl, iso-Butyl, sec-Butyl, oder tert-Butylbedeutet, und R'-OH ebenfalls abdestilliert wird.

6. Verfahren nach dem vorhergehenden Anspruch, **dadurch gekennzeichnet, dass** für den Fall, dass ein Alkalimetallalkoholat eingesetzt wird, der Rest R' dem Rest R entspricht.

7. Verfahren nach einem der beiden vorhergehenden Ansprüche, **dadurch gekennzeichnet, dass** der Gehalt der Alkalimetallverbindung in der Lösung 1 bis 50 Gew.-% (bezogen auf die Gesamtmasse der Lösung) beträgt.

8. Verfahren nach einem der drei vorhergehenden Ansprüche, **dadurch gekennzeichnet, dass** als Alkalimetallverbindung Natriummethylat (20 bis 40 Gew.-%ig in Methanol) oder Kaliumethylat (15 bis 35 Gew.-%ig in Ethanol) eingesetzt wird.

9. Verfahren nach einem der vorhergehenden Ansprüche, **dadurch gekennzeichnet, dass** das roh-Morpholin erhalten wird durch Umsetzung von Diethylenglykol (DEG) mit Ammoniak und anschließender destillativer Abtrennung von Ammoniak, Wasser, Aminodiglykol (ADG) und DEG aus dem Umsetzungsprodukt.

10. Verfahren nach einem der vorhergehenden Ansprüche, **dadurch gekennzeichnet, dass** das roh-Morpholin > 85 Gew.-% Morpholin (MO) und 0,1 bis 5 Gew.-% Methoxyethanol (MOE) enthält.

11. Verfahren nach einem der vorhergehenden Ansprüche, **dadurch gekennzeichnet, dass** das abdestillierte Morpholin (MO) eine Reinheit von > 98,5 Gew.-% und einen Gehalt an Methoxyethanol (MOE) von < 0,1 Gew.-% hat.

12. Verfahren nach einem der vorhergehenden Ansprüche, **dadurch gekennzeichnet, dass** der Kopfdruck in der Destillationskolonne 0,01 bis 12 bar beträgt.

13. Verfahren nach einem der vorhergehenden Ansprüche, **dadurch gekennzeichnet, dass** bei einer kontinuierlichen Fahrweise roh-Morpholin und die Alkalimetallverbindung der Destillationskolonne zugeführt werden, R-OH und gegebenenfalls R'-OH über Kopf und MO über einen Seitenabzug abgeführt werden und über Sumpf Alkalimetallmethoxyethanolat ausgeschleust wird.

14. Verfahren nach dem vorhergehenden Anspruch, **dadurch gekennzeichnet, dass** sich die Zuführung der Alkalimetallverbindung und der Seitenabzug, an dem MO abgeführt wird, im Abtriebsteil der Destillationskolonne befinden.

15. Verfahren nach dem vorhergehenden Anspruch, **dadurch gekennzeichnet, dass** sich die Zuführung der Alkalimetallverbindung über dem Seitenabzug befindet, an dem MO abgeführt wird.

16. Verfahren nach dem vorhergehenden Anspruch, **dadurch gekennzeichnet, dass** die Position der Zuführung der Alkalimetallverbindung im Abtriebsteil der Kolonne so gewählt ist, dass dort keine signifikante Menge an Wasser vorhanden ist.

## Claims

1. A process for the depletion of 2-methoxyethanol (MOE) from a mixture comprising predominantly morpholine (MO) (crude morpholine), wherein crude morpholine is distilled in a distillation column in the presence of an alkali metal compound of the general formula M⁺[RO⁻] (M⁺ is alkali metal cation and R is hydrogen (H), methyl, ethyl, propyl, isopropyl, n-butyl, isobutyl, sec-butyl or tert-butyl), where MO and a compound of the general formula R-OH are distilled off and an alkali metal methoxyethoxide of the general formula M⁺[MeOEtO⁻] is obtained in the bottom of the column.

2. The process according to claim 1, wherein the alkali metal compound is used in a 0.1-fold to 5-fold molar amount based,
(a) when an alkali metal hydroxide (alkali metal compound in which R is hydrogen (H)) is used, on the MOE (2-methoxyethanol) present in the crude morpholine,
(b) when an alkali metal alkoxide (alkali metal compound in which R is not hydrogen (H)) is used,
■ in a batchwise mode of operation, on the MOE (2-methoxyethanol) present in the crude morpholine and optionally water present in the crude morpholine, or
■ in a continuous mode of operation, on the MOE (2-methoxyethanol) present in the crude morpholine.

3. The process according to either of the preceding claims, wherein M⁺ is Li⁺, Na⁺ or K⁺.

4. The process according to any of the preceding claims, wherein the alkali metal compound is selected from the group consisting of lithium hydroxide, sodium hydroxide, potassium hydroxide, lithium methoxide, sodium methoxide, potassium methoxide, lithium ethoxide, sodium ethoxide, potassium ethoxide, lithium propoxide, sodium propoxide, potassium propoxide, lithium isopropoxide, sodium isopropoxide, potassium isopropoxide, lithium n-butoxide, sodium n-butoxide, potassium n-butoxide, lithium isobutoxide, sodium isobutoxide, potassium isobutoxide, lithium sec-butoxide, sodium sec-butoxide, potassium sec-butoxide, lithium tert-butoxide, sodium tert-butoxide, and potassium tert-butoxide.

5. The process according to any of the preceding claims, wherein the alkali metal compound is used in the form of a solution in an alcohol of the general formula R'-OH, in which R' is methyl, ethyl, propyl, isopropyl, n-butyl, isobutyl, sec-butyl or tert-butyl, and R'-OH is likewise distilled off.

6. The process according to the preceding claim, wherein in the case where an alkali metal alkoxide is used, the radical R' corresponds to the radical R.

7. The process according to either of the two preceding claims, wherein the content of the alkali metal compound in the solution is 1% to 50% by weight (based on the total mass of the solution).

8. The process according to any of the three preceding claims, wherein the alkali metal compound used is sodium methoxide (20% to 40% by weight in methanol) or potassium ethoxide (15% to 35% by weight in ethanol).

9. The process according to any of the preceding claims, wherein the crude morpholine is obtained by reaction of diethylene glycol (DEG) with ammonia and subsequent distillative removal of ammonia, water, aminodiglycol (ADG) and DEG from the reaction product.

10. The process according to any of the preceding claims, wherein the crude morpholine comprises > 85% by weight of morpholine (MO) and 0.1% to 5% by weight of methoxyethanol (MOE).

11. The process according to any of the preceding claims, wherein the morpholine (MO) distilled off has a purity of > 98.5% by weight and a content of methoxyethanol (MOE) of < 0.1% by weight.

12. The process according to any of the preceding claims, wherein the top pressure in the distillation column is 0.01 to 12 bar.

13. The process according to any of the preceding claims, wherein for a continuous mode of operation crude morpholine and the alkali metal compound are supplied to the distillation column, R-OH and optionally R'-OH are removed overhead and MO is removed via a side draw, and alkali metal methoxyethoxide is discharged via the bottom.

14. The process according to the preceding claim, wherein the feed for the alkali metal compound and the side draw at which MO is removed are located in the stripping section of the distillation column.

15. The process according to the preceding claim, wherein the feed for the alkali metal compound is located above the side draw at which MO is removed.

16. The process according to the preceding claim, wherein the position of the feed for the alkali metal compound in the stripping section of the column is selected such that there is no significant amount of water present there.

## Revendications

1. Procédé d'appauvrissement de 2-méthoxyéthanol (MOE) d'un mélange contenant majoritairement de la morpholine (MO) (morpholine brute), **caractérisé en ce que** de la morpholine brute est distillée dans une colonne de distillation en présence d'un composé de métal alcalin de formule générale M⁺[RO⁻1 (M⁺ signifie un cation de métal alcalin et R signifie hydrogène (H), méthyle, éthyle, propyle, iso-propyle, n-butyle, iso-butyle, sec-butyle ou tert-butyle), MO ainsi qu'un composé de formule générale R-OH étant distillés et dans le fond de la colonne un méthoxyéthanolate de métal alcalin de formule générale M⁺[MeOEtO⁻] étant précipité.

2. Procédé selon la revendication 1, **caractérisé en ce que** le composé de métal alcalin est utilisé en la quantité molaire de 0,1 à 5 fois par rapport
(a) au MOE (2-méthoxyéthanol) contenu dans la morpholine brute, lorsqu'un hydroxyde de métal alcalin (composé de métal alcalin dans lequel R signifie hydrogène (H)) est utilisé,
(b) lorsqu'un alcoolate de métal alcalin (composé de métal alcalin dans lequel R ne signifie pas hydrogène (H)) est utilisé,
• lors d'un mode de fonctionnement de manière discontinue, au MOE (2-méthoxyéthanol) contenu dans la morpholine brute et éventuellement à l'eau contenue dans la morpholine brute, ou
• lors d'un mode de fonctionnement de manière continue, au MOE (2-méthoxyéthanol) contenu dans la morpholine brute.

3. Procédé selon l'une quelconque des revendications précédentes, **caractérisé en ce que** M⁺ signifie Li⁺, Na⁺ ou K⁺.

4. Procédé selon l'une quelconque des revendications précédentes, **caractérisé en ce que** le composé de métal alcalin est choisi dans le groupe constitué par l'hydroxyde de lithium, l'hydroxyde de sodium, l'hydroxyde de potassium, le méthanolate de lithium, le méthanolate de sodium, le méthanolate de potassium, l'éthanolate de lithium, l'éthanolate de sodium, l'éthanolate de potassium, le propanolate de lithium, le propanolate de sodium, le propanolate de potassium, l'isopropanolate de lithium, l'isopropanolate de sodium, l'isopropanolate de potassium, le n-butanolate de lithium, le n-butanolate de sodium, le n-butanolate de potassium, l'iso-butanolate de lithium, l'iso-butanolate de sodium, l'iso-butanolate de potassium, le sec-butanolate de lithium, le sec-butanolate de sodium, le sec-butanolate de potassium, le tert-butanolate de lithium, le tert-butanolate de sodium et le tert-butanolate de potassium.

5. Procédé selon l'une quelconque des revendications précédentes, **caractérisé en ce que** le composé de métal alcalin est utilisé en tant que solution dans un alcool de formule générale R'-OH, dans laquelle R' signifie méthyle, éthyle, propyle, iso-propyle, n-butyle, iso-butyle, sec-butyle ou tert-butyle, et R'-OH est également distillé.

6. Procédé selon la revendication précédente, **caractérisé en ce que** dans le cas où un alcoolate de métal alcalin est utilisé, le radical R' correspond au radical R.

7. Procédé selon l'une quelconque parmi les deux revendications précédentes, **caractérisé en ce que** la teneur du composé de métal alcalin dans la solution est de 1 à 50 % en poids (par rapport à la masse totale de la solution).

8. Procédé selon l'une quelconque parmi les trois revendications précédentes, **caractérisé en ce que** du méthylate de sodium (solution à 20 à 40 % en poids dans le méthanol) ou de l'éthylate de potassium (solution à 15 à 35 % en poids dans l'éthanol) est utilisé en tant que composé de métal alcalin.

9. Procédé selon l'une quelconque des revendications précédentes, **caractérisé en ce que** la morpholine brute est obtenue par transformation de diéthylèneglycol (DEG) avec de l'ammoniac et ensuite séparation par distillation d'ammoniac, d'eau, d'aminodiglycol (ADG) et de DEG à partir du produit de transformation.

10. Procédé selon l'une quelconque des revendications précédentes, **caractérisé en ce que** la morpholine brute contient > 85 % en poids de morpholine (MO) et 0,1 à 5 % en poids de méthoxyéthanol (MOE).

11. Procédé selon l'une quelconque des revendications précédentes, **caractérisé en ce que** la morpholine (MO) distillée possède une pureté de > 98,5 % en poids et une teneur en méthoxyéthanol (MOE) de < 0,1 % en poids.

12. Procédé selon l'une quelconque des revendications précédentes, **caractérisé en ce que** la pression de tête dans la colonne de distillation est de 0,01 à 12 bars.

13. Procédé selon l'une quelconque des revendications précédentes, **caractérisé en ce que** lors d'un mode de fonctionnement de manière continue, de la morpholine brute et le composé de métal alcalin sont alimentés à la colonne de distillation, R-OH et éventuellement R'-OH sont soutirés par la tête et MO est soutiré par le biais d'un soutirage latéral et le méthoxyéthanolate de métal alcalin est évacué par le fond.

14. Procédé selon la revendication précédente, **caractérisé en ce que** l'alimentation de composé de métal alcalin et le soutirage latéral par lequel MO est soutiré, se trouvent dans la partie de sortie de la colonne de distillation.

15. Procédé selon la revendication précédente, **caractérisé en ce que** l'alimentation de composé de métal alcalin se trouve au-dessus du soutirage latéral par lequel MO est soutiré.

16. Procédé selon la revendication précédente, **caractérisé en ce que** la position de l'alimentation de métal alcalin dans la partie de sortie de la colonne est choisie de telle manière qu'aucune quantité significative d'eau ne se trouve là.
